# EUROPÄISCHE PATENTSCHRIFT

(11) **EP 3 044 576 B1**
(45) Veröffentlichungstag und Bekanntmachung des Hinweises auf die Patenterteilung: **19.07.2017**
(21) Anmeldenummer: 14758785.1
(22) Anmeldetag: 01.09.2014
(51) Int. Cl.: G01N 27/404, G01N 33/00

(54) **ELEKTROCHEMISCHER GASSENSOR, FLÜSSIGER ELEKTROLYT UND VERWENDUNG EINES FLÜSSIGEN ELEKTROLYTEN**
ELECTROCHEMICAL GAS SENSOR, LIQUID ELECTROLYTE AND USE OF A LIQUID ELECTROLYTE
CAPTEUR DE GAZ ÉLECTROCHIMIQUE, ÉLECTROLYTE LIQUIDE ET UTILISATION D'UN ÉLECTROLYTE LIQUIDE

(30) Priorität: 09.09.2013 DE 102013014994
(43) Veröffentlichungstag der Anmeldung: 20.07.2016
(73) Patentinhaber: Dräger Safety AG & Co. KGaA, 23560 Lübeck (DE)
(72) Erfinder: NAUBER, Andreas, 23617 Stockelsdorf (DE); SICK, Michael, 23669 Timmendorfer Strand (DE); STEINER, Gregor, 79822 Titisee-Neustadt (DE); MATTERN-FRÜHWALD, Marie-Isabell, 22941 Bargteheide (DE); METT, Frank, 23556 Lübeck (DE); CHRZAN, Rigobert, 23843 Bad Oldesloe (DE); SOMMER, Sabrina, 23558 Lübeck (DE)
(74) Vertreter: Kettenbeil, Roxane Henriette
(86) Internationale Anmeldenummer: PCT/EP2014/002363
(87) Internationale Veröffentlichungsnummer: WO 2015/032481

(56) Entgegenhaltungen:
- EP-A1- 2 224 018
- WO-A1-2013/045561
- WO-A1-2013/060773
- GB-A- 2 225 859
- US-A- 5 746 900
- US-A1- 2005 202 982
- XIAOBO JI ET AL: "Determination of ammonia based on the electrochemical oxidation of N,N'-diphenylenediamine in propylene carbonate", ANALYTICAL SCIENCES, Bd. 23, November 2007 (2007-11), Seiten 1317-1320, XP055154055,

## Beschreibung

Die Erfindung betrifft einen elektrochemischen Gassensor, sowie die Verwendung eines solchen Gassensors zum Nachweis von NH₃ oder NH₃-haltigen Gasgemischen Elektrochemische Gassensoren, mit denen über einen begrenzten Zeitraum die Konzentration von gasförmigen Stickstoffverbindungen detektierbar ist, sind allgemein bekannt. Solche Sensoren kommen üblicherweise in den verschiedensten technischen Bereichen zum Einsatz, von der chemischen Industrie über die Überwachung von Kühlanlagen bis hin zu landwirtschaftlichen Betrieben. Sie dienen insbesondere dazu, kritische Konzentrationen von entzündlichen und/oder giftigen Gasen rechtzeitig zu erkennen und vor einer entsprechenden Gefahr zu warnen, Insbesondere die Überwachung der Konzentration von Ammoniak (NH₃), Hydrazin und Aminen ist dabei von Interesse. Solche elektrochemischen Sensoren bestehen üblicherweise aus mehreren Elektroden, die in leitendem Kontakt mit einer Elektrolytflüssigkeit stehen und auf diese Weise ein galvanisches Element - im Folgenden auch elektrochemische Messzelle genannt - bilden.

EP 0 395 927 B1 offenbart beispielsweise eine elektrochemische Messzelle zur Bestimmung von Ammoniak oder Hydrazin in einer gasförmigen oder flüssigen Messprobe, mit mindestens einer Messelektrode und einer Gegenelektrode. Um für die Bestimmung von Ammoniak oder Hydrazin ein Referenzpotential zu erzeugen, wird in diese Messzelle eine Referenzelektrode eingebracht, deren Potential als Bezugspunkt für die Messung dient. Auch EP 0 556 558 B1 offenbart eine solche elektrochemische Messzellen zur Bestimmung von Ammoniak, Aminen, Hydrazin und Hydrazinderivaten.

Der Nachweis der stickstoffhaltigen Verbindungen, z.B. Ammoniak, verschiedene Aminen oder Hydrazin, erfolgt bei solchen Messzellen typischerweise mit Hilfe einer elektrochemischen Reaktion zwischen dem in den Sensor einströmenden Gas, den Elektroden und dem Elektrolyten des Sensors. Beispielsweise kann einströmendes Ammoniak an einer ersten Elektrode (typischerweise als Arbeitselektrode bezeichnet) oxidiert werden. Dabei können sich Ammoniumionen bilden, die durch den Elektrolyten zu einer zweiten Elektrode (typischerweise als Gegenelektrode bezeichnet) diffundieren. Dort können die Ammoniumionen wieder deprotoniert werden. Diese Reaktion führt zu einem nachweisbaren Stromfluss in der galvanischen Zelle. Der Stromfluss zeigt damit die Anwesenheit des nachzuweisenden Gases (im Folgenden auch als "reaktive Spezies" bezeichnet) an.

Bei solcherart bekannten elektrochemischen Messzellen können jedoch verschiedene Probleme auftreten. So können sich bei der oben beschriebenen Reaktion nicht nur Ammoniumionen, sondern auch weitere Stickstoffverbindungen bilden. Diese können sich jedoch an den Elektroden anlagern und auf diese Weise die Reaktion von weiteren in den Sensor eintretenden Ammoniakmolekülen oder anderen nachzuweisenden Molekülen erschweren bis hin zu einer nahezu vollständigen Blockierung der Reaktion. Man spricht in diesem Zusammenhang auch von einer Vergiftung des Sensors (Sensorvergiftung). Eine solche Sensorvergiftung kann einerseits dazu führen, dass sich die grundsätzliche Messempfindlichkeit des Sensors verschlechtert, andererseits kann die Signalstabilität bei Dauerbegasung deutlich absinken. Mit jeder Detektion von gasförmigem Ammoniak kann dabei die Empfindlichkeit des Sensors weiter abnehmen, bis schließlich keine zuverlässige Messung mehr möglich ist. Daneben können auch Änderungen des Nullsignals bei einer Änderung der Umgebungsfeuchte und die Querempfindlichkeit auf andere Gase problematisch sein.

Ein weiterer elektrochemischer Gassensor ist aus der US 5, 746,900 A bekannt. Dieser Gassensor hat ein Gehäuse, eine Arbeits-, eine Gegen- und eine Referenzelektrode. Das Gehäuse hat ein Elektrolytreservoir und einen Gegenelektrodenträger, auf dem die ringförmig ausgebildete Gegenelektrode vollflächig aufliegt.

Auch WO 2013/060773 A1 offenbart einen elektrochemischen Gassensor mit einem Gehäuse und drei Elektroden. Dieser Sensor hat jedoch keinen Gegenelektrodenträger Gleiches gilt für die Schriften GB 225 859 A, EP 2 224 018 A1, WO 2013/045561 A1 und Xiaobo et al ("Determination of ammonia based on the electrochemical oxidation of N,N'-diphenylenediamine in propylene carbonate", Analytical Sciences, Bd. 23, Nov. 2007, Seiten 1317-1320).

Ausgehend davon ist es Aufgabe der vorliegenden Erfindung, diese und weitere Nachteile des Standes der Technik zu überwinden und einen verbesserten elektrochemischen Gassensor bereit zu stellen. Insbesondere soll ein Gassensor bereitgestellt werden, der eine möglichst hohe Messempfindlichkeit, eine möglichst gute Signalstabilität bei Dauerbelastung und/oder eine soweit wie möglich verringerte Querempfindlichkeit aufweist. Weiterhin soll der Gassensor möglichst kostengünstig und einfach herstellbar sein.

Als Lösung sieht die Erfindung einen elektrochemischen Gassensor mit den Merkmalen des Anspruchs 1, sowie die Verwendung eines solchen Gassensors zum Nachweis von NH₃ oder NH₃-haltigen Gasgemischen entsprechend Anspruch 15 vor. Weitere Ausgestaltungen sind Gegenstand der jeweils abhängigen Ansprüche. Bei einem elektrochemischer Gassensor mit einem Gehäuse, einer Arbeitselektrode, einer Gegenelektrode und einer Referenzelektrode, wobei das Gehäuse ein Elektrolytreservoir, eine Gaseintrittsöffnung und wenigstens eine Gasaustrittsöffnung aufweist und das Elektrolytreservoir mit einem flüssigen Elektrolyten gefüllt ist, sieht die Erfindung vor, dass der Gassensor einen Gegenelektrodenträger aufweist, wobei die Gegenelektrode derart an dem Gegenelektrodenträger aufgehängt ist, dass sie im Elektrolytreservoir hängt und von allen Seiten von dem Elektrolyt umspült ist.

Bei einem solchen Sensor ist es von großem Vorteil, dass der Gassensor einen Gegenelektrodenträger aufweist, an welchem die Gegenelektrode aufgehängt ist. Der Elektrolyt kann auf diese Weise die Gegenelektrode von allen Seiten umspülen. Auf diese Weise können einerseits zur Gegenelektrode hin diffundierende Ionen, die bei der Reaktion der reaktiven Spezies an der Arbeitselektrode entstehen - beispielsweise Ammoniumionen, frei durch den Elektrolyten zur Gegenelektrode gelangen. Andererseits können auch durch die Rückreaktion an der Gegenelektrode entstehende reaktive Spezies - beispielsweise Ammoniakmoleküle - oder andere Reaktionsprodukte wieder von der Gegenelektrode abtransportiert werden. In diesem Zusammenhang ist es auch von Vorteil, dass der elektrochemische Gassensor eine Gasaustrittsöffnung aufweist. Das nachzuweisende Gas kann so nach erfolgter Rückreaktion an der Gegenelektrode auf einfache Weise wieder aus dem Gassensor freigesetzt werden. Zum zusätzlichen Druckausgleich kann auch eine weitere Öffnung in der seitlichen Gehäusewand vorhanden sein. Das an der Gegenelektrode entstehende Gas kann mithin durch die Gasaustrittsöffnung und durch die weitere Öffnung entweichen, so dass es weder zu einer Vergiftung der Gegenelektrode noch zu einem unerwünschten Überdruck im Sensor kommt.

Man erkennt mithin, dass die Gegenelektrode bevorzugt keine Gasdiffusionselektrode ist. Vielmehr kann die Gegenelektrode zum Beispiel eine drahtförmige Elektrode sein, die von dem Gegenelektrodenträger in den Elektrolyten herab hängen kann.

Die Arbeitselektrode ist bevorzugt eine Gasdiffusionselektrode, die in dem Gehäuse hinter der Gaseintrittsöffnung angeordnet ist. Beispielsweise ist es günstig, wenn die Arbeitselektrode derart in dem Gehäuse angeordnet ist, dass das durch den Gaseinlass einströmende Gas direkt auf die Arbeitselektrode trifft. Vorstellbar ist dabei, dass zwischen dem Gaseinlass und der Arbeitselektrode eine Schutzmembran angeordnet ist, die verhindert, dass die Arbeitselektrode mechanisch, z.B. durch Staubteilchen, beschädigt werden kann.

Die Arbeitselektrode kann beispielsweise eine beschichtete PTFE-Membran sein. Die Beschichtung kann beispielsweise eine Beschichtung aus Kohlenstoffnanoröhren sein. Vorstellbar ist jedoch auch, dass die Arbeitselektrode aus einem Edelmetall oder einem Edelmetallgemisch besteht. So ist es unter anderem denkbar, dass die Arbeitselektrode eine Kohlenstoffelektrode, bevorzugt eine Elektrode aus einwandigen Kohlenstoffnanoröhren, mehrwandigen Kohlenstoffananoröhren oder oberflächenaktivem Kohlenstoff, oder eine Iridium-Sputter-Elektrode ist. Beispielsweise kann die Arbeitselektrode auch eine PTFE-Membran sein, die mit Iridium oder einem anderen Metall besputtert wurde.

Vorteilhafterweise besteht dabei die Gegenelektrode ganz oder teilweise aus einwandigen Kohlenstoffnanoröhren, mehrwandigen Kohlenstoffannoröhren, oberflächenaktivem Kohlenstoffe, Ruthenium, Iridium, Platin, Palladium, Gold oder Mischungen aus Ruthenium, Iridium, Platin, Palladium und/oder Gold. Dabei können die Arbeitselektrode und/oder die Gegenelektrode aus Edelmetall, Edelmetallgemisch oder Kohlenstoff bestehen. Die Arbeitselektrode und die Gegenelektrode können in diesem Zusammenhang aus dem gleichen Material bestehen. Alternativ können die Arbeitselektrode und die Gegenelektrode auch aus unterschiedlichen Materialien bestehen.

In einer bevorzugten Ausführungsform ist es vorteilhaft, wenn der Gassensor ein Trennelement aufweist, welches das Gehäuse in eine obere Kammer und eine untere Kammer unterteilt. Dabei sind sowohl in der oberen Kammer als auch in der unteren Kammer Elektroden angeordnet. Es ist daher günstig, wenn sowohl in der oberen Kammer als auch in der unteren Kammer Elektrolyt vorhanden ist. Bevorzugt sind die obere Kammer und die untere Kammer miteinander strömungsverbunden, so dass ein Austausch zwischen dem Elektrolyten in der oberen Kammer und dem Elektrolyten in der unteren Kammer stattfinden kann.

Besonders bevorzugt ist es dabei, wenn die obere Kammer das Elektrolytreservoir bildet. Mithin ist es sinnvoll, wenn die Gegenelektrode in der oberen Kammer angeordnet ist. Die Arbeitselektrode ist auch hier zum Beispiel eine Gasdiffusionselektrode, die wie oben beschrieben in dem Gehäuse hinter der Gaseintrittsöffnung angeordnet ist. Dabei ist es günstig, wenn die Arbeitselektrode in der unteren Kammer angeordnet ist. Zwischen der Arbeitselektrode und dem Trennelement kann dabei eine Zwischenmembran angeordnet sein. Die Zwischenmembran kann zum einen die Arbeitselektrode vor einer Beschädigung durch einen direkten Kontakt mit dem Trennelement schützen. Dies ist beispielweise dann sinnvoll, wenn das Trennelement nicht nur dazu dient, das Gehäuse in zwei Kammern zu unterteilen, sondern auch dazu, die Arbeitselektrode in der unteren Kammer in Richtung des Gaseinlass zu drücken. Zum anderen kann die Zwischenmembran dazu dienen, den Elektrolyten von der oberen Kammer in die untere Kammer zu leiten. Beispielsweise kann der Elektrolyt durch eine Öffnung im Trennelement von der oberen Kammer in die untere Kammer leitbar sein. Die Zwischenmembran kann dann einen gewissen Mindestabstand zwischen dem Trennelement und der darunter angeordneten Elektrode sicherstellen. Die Zwischenmembran ist bevorzugt derart ausgebildet, dass sie für den Elektrolyten durchlässig ist. Gleichzeitig ist die Zwischenmembran günstigerweise derart formstabil, dass sie einen gewissen Mindestabstand zwischen denjenigen Bauteilen gewährleisten kann, zwischen denen sie angeordnet ist. Beispielsweise kann die Zwischenmembran eine Glasfasermembran sein. Auf diese Weise kann der Elektrolyt, der durch das Trennelement hindurch von der oberen Kammer zur unteren Kammer strömt durch die Zwischenmembran hindurch in die untere Kammer gelangen. Die Arbeitselektrode kann auf diese Weise stets mit Elektrolyt umspült sein. Außerdem kann so stets ein Fluidkontakt zwischen dem Elektrolyten in der oberen Kammer und der unteren Kammer bestehen. Insbesondere kann mit Hilfe des Elektrolyten auf diese Weise stets ein leitender Kontakt zwischen der Arbeitselektrode und der Gegenelektrode bestehen. Dies ist insbesondere dann vorteilhaft, wenn die Arbeitselektrode in der unteren Kammer und die Gegenelektrode wie oben beschrieben in der oberen Kammer angeordnet ist. Insofern erkennt man in diesem Zusammenhang, dass es vorteilhaft ist, wenn das Trennelement derart ausgebildet ist, dass Elektrolyt durch das Trennelement hindurch aus der oberen Kammer in die untere Kammer leitbar ist.

Vorstellbar ist dabei, dass das Trennelement aus einem für den Elektrolyten grundsätzlich durchlässigen Material besteht. Das Trennelement kann in diesem Fall insbesondere dazu dienen, einen gewissen Mindestabstand zwischen der in der unteren Kammer angeordneten Arbeitselektrode und der in der oberen Kammer angeordneten Gegenelektrode zu gewährleisten. Dazu kann das Trennelement derart ausgestaltet sein, dass es die Arbeitselektrode möglichst weit in Richtung des Gaseinlasses drückt.

In einer bevorzugten Ausführungsform ist es auch vorstellbar, dass das Trennelement grundsätzlich aus einem für den Elektrolyten nicht durchlässigen Material besteht. In dem Trennelement kann dann zum Beispiel eine Durchlassöffnung ausgebildet sein, die zum Austausch von Elektrolyt zwischen der oberen und der unteren Kammer dient. Die Durchlassöffnung kann beispielsweise ein Durchlasskanal sein.

Bevorzugt ist es dabei etwa, wenn das Trennelement wenigstens ein Oberteil und ein Fußteil aufweist. Dabei kann das Fußteil dazu dienen, die obere Kammer von der unteren Kammer zu trennen. Weiterhin kann das Fußteil die Arbeitselektrode und - sofern vorhanden - die zwischen dem Trennelement und der Arbeitselektrode angeordnete Zwischenmembran in Richtung auf den Gaseinlass im Gehäuse festzuhalten. Das Oberteil kann beispielsweise dochtartig ausgebildet sein und dazu dienen den Elektrolyten von der oberen Kammer in die untere Kammer zu leiten. Beispielsweise kann das Oberteil die Form eines Rohres haben. Dieses Rohr hat bevorzugt eine obere und eine untere Öffnung. Dabei ist das Rohr ist beispielsweise derart auf dem Fußteil angebracht, dass die obere Öffnung zur oberen Kammer hin offen ist und die innere Öffnung des Rohres mit einer Öffnung im Fußteil korrespondiert. Der Elektrolyt aus der oberen Kammer kann auf diese Weise durch die obere Öffnung in das Rohr gelangen und von dort durch die untere Öffnung in die untere Kammer. Auf diese Weise kann sichergestellt werden, dass zwischen der oberen Kammer und der unteren Kammer eine Fluidverbindung besteht.

Denkbar ist auch, dass der Gegenelektrodenträger ein Teil des Trennelementes ist. Beispielsweise kann der Gegenelektrodenträger am Oberteil des Trennelementes angebracht sein. Man erkennt, dass die Gegenelektrode bevorzugt in der oberen Kammer angeordnet ist.

In einer weiteren bevorzugten Ausgestaltung ist es vorteilhaft, wenn der Gassensor außerdem eine Abfangelektrode aufweist. Eine solche Abfangelektrode kann verhindern, dass ein Überschuss der nachzuweisenden reaktiven Spezies unkontrolliert in die obere Kammer diffundiert. Bevorzugt ist die Abfangelektrode dabei zwischen der Arbeitselektrode und dem Trennelement angeordnet.

Dabei ist es vorstellbar, dass auch zwischen der Arbeitselektrode und der Abfangelektrode eine Zwischenmembran angeordnet ist. Eine solche Zwischenmembran ist bevorzugt auch hier aus einem für den Elektrolyten durchlässigen Material, das zu einem gewissen Grad formstabil ist, beispielsweise eine Glasfasermembran. Die Abfangelektrode und die Arbeitselektrode können auf diese Weise derart angeordnet sein, dass sie nicht in direktem Kontakt miteinander stehen, wobei gleichzeitig ein elektrisch leitender Kontakt durch den Elektrolyten besteht, welcher die Zwischenmembran tränkt. Mit Hilfe dieser Zwischenmembran, kann der Abstand zwischen der Arbeitselektrode und der Abfangelektrode dabei relativ gering bemessen sein und wird lediglich durch die Dicke der Zwischenmembran bestimmt.

Auch zwischen der Abfangelektrode und dem Trennelement kann eine Zwischenmembran angeordnet sein. Diese Zwischenmembran funktioniert dann wie die bereits oben beschriebene Zwischenmembran, die - im Falle einer nicht vorhandenen Abfangelektrode - zwischen der Arbeitselektrode und dem Trennelement angeordnet ist.

Man erkennt, dass ein erfindungsgemäßer elektrochemischer Gassensor beispielsweise derart aufgebaut sein kann, dass er ein Gehäuse aufweist, welches mit Hilfe eines Trennelementes in eine obere Kammer und eine untere Kammer unterteilt ist. Die obere Kammer kann dabei als Elektrolytreservoir dienen. Die obere und die untere Kammer des Gehäuses können mit einem flüssigen Elektrolyten gefüllt sein. Dabei ist das Trennelement bevorzugt derart ausgebildet, dass der Elektrolyt durch das Trennelement hindurch von der oberen Kammer in die untere Kammer strömen kann.

In der unteren Kammer kann ein Gaseinlass ausgebildet sein, in der oberen Kammer ein Gasauslass. Weiterhin kann in der oberen Kammer eine zusätzliche Öffnung zum Druckausgleich ausgebildet sein. Durch den Gaseinlass kann das nachzuweisende Gas - d.h. die reaktive Spezies - in das Gehäuse einströmen.

Bevorzugt ist dabei die Arbeitselektrode des erfindungsgemäßen Gassensors in Strömungsrichtung hinter dem Gaseinlass in der unteren Kammer angeordnet. Gunstigerweise ist dabei zwischen dem Gaseinlass und der Arbeitselektrode eine Schutzmembran ausgebildet. In der unteren Kammer kann weiterhin bevorzugt eine Abfangelektrode ausgebildet sein. Die Arbeitselektrode und die Abfangelektrode können dabei durch eine Zwischenmembran, beispielsweise eine Glasfasermembran, voneinander getrennt sein. Eine weitere Zwischenmembran ist bevorzugt zwischen der Abfangelektrode und dem Trennelement ausgebildet. Ist keine Abfangelektrode vorhanden, so kann auch zwischen der Arbeitselektrode und dem Trennelemente eine Zwischenmembran ausgebildet sein. Man erkennt, dass es günstig ist, wenn ein erfindungsgemäßer Gassensor wenigstens eine Zwischenmembran zwischen der Arbeitselektrode und dem Trennelement aufweist. Bevorzugt weist ein erfindungsgemäßer Gassensor eine erste Zwischenmembran zwischen der Arbeitselektrode und der Abfangelektrode und eine zweite Zwischenmembran zwischen der Abfangelektrode und dem Trennelement auf.

Die Gegenelektrode ist bei einem solchen erfindungsgemäßen Gassensor bevorzugt in der oberen Kammer, mithin im Elektrolytreservoir, angeordnet. Dabei weist der Gassensor einen Gegenelektrodenträger auf, an welchem die Gegenelektrode befestigt ist. Der Gegenelektrodenträger ist bevorzugt ein Teil des wie oben beschrieben ausgebildeten Trennelementes.

Vorteilhaft ist es weiterhin, wenn die Referenzelektrode in dem Elektrolytreservoir angeordnet ist und von allen Seiten von dem Elektrolyt umspült ist. Dabei ist es günstig, wenn die Referenzelektrode an dem Gegenelektrodenträger aufgehängt ist. Die Referenzelektrode kann auf diese Weise einen möglichst großen Abstand von den übrigen Elektroden aufweisen aber gleichzeitig effektiv und genau die Verhältnisse im Sensor erfassen.

Zum Nachweis von reaktiven Spezies, die beispielsweise Amin-Verbindungen, Ammoniak oder Hydrazin umfassen, ist es besonders bevorzugt, wenn der Elektrolyt wenigstens ein Lösungsmittel, ein Leitsalz und/oder einen organischen Mediator enthält.

Dabei ist es in jedem Fall günstig, wenn das Lösungsmittel ausgewählt ist aus der Gruppe enthaltend Wasser und Alkylencarbonat oder Gemische daraus, bevorzugt ausgewählt aus der Gruppe enthaltend Wasser, Propylencarbonat, Ethylencarbonat oder Gemische daraus. Besonders günstig hat sich dabei ein Gemisch aus Propylencarbonat und Ethylencarbonat erwiesen. Dabei ist es weiterhin günstig, wenn das Leitsalz eine ionische Flüssigkeit, ein anorganisches Salz oder ein Gemisch aus einer ionischen Flüssigkeit und einem anorganischen Salz ist, wobei das Anion bevorzugt ausgewählt ist aus der Gruppe enthaltend Halogenide, Carbonat, Sulfonat, Phosphat und/oder Phosphonat, und wobei das Kation bevorzugt ausgewählt ist aus der Gruppe enthaltend Metallionen, Oniumionen oder ein Gemisch aus Metallionen und Oniumionen Unter dem Begriff Oniumionen werden dabei Kationen verstanden, die formal durch die Protonierung eines mononucleären Ausgangshydrids von Elementen der Stickstoff-, Kohlenstoff- oder Halogengruppe erhalten werden können.

Weiterhin ist es günstig, wenn der organische Mediator ein chinoides System aufweist, bevorzugt ausgewählt aus der Gruppe enthaltend ortho-Chinone, para-Chinone, substituierte ortho-Chinone und substituierte para-Chinone, Dihydroxynaphatlin, substitulertes Dihydroxynaphtalin, Anthrachinon, substituiertes Anthrachinon, besonders bevorzugt ausgewählt aus der Gruppe enthaltend 1,2-Dihydroxybenzol, 1,4-Dihydroxybenzol, 1,4-Naphtohydrochinon, substituiertes 1,2-Dihydroxybenzol, substituiertes 1,4-Dihydroxybenzol, substituiertes 1,4-Naphtohydrochinon, ganz besonders bevorzugt ausgewählt aus der Gruppe enthaltend substituiertes Anthrachinon, substituiertes 1,2-Dihydroxybenzol, subsituiertes 1,4-Dihydroxybenzol. Besonders bevorzugt ist der organische Mediator dabei eine Dihydroxybenzol-Verbindung, die an der Arbeitselektrode zu einer Chinon-Verbindung oxidiert werden kann. Dabei kann gleichzeitig in den Sensor einströmendes Gas - z.B. Ammoniak, eine Amin-Verbindung oder auch Hydrazin - reduziert werden. Im Zuge der Gegenreaktion an der Gegenelektrode kann die entstandene Chinon-Verbindung wieder zur Dihydroxybenzol-Verbindung reduziert werden und das Gas - beispielsweise bei der Hinreaktion entstandene Ammoniumionen - kann wieder zu seinem Ausgangszustand oxidiert und anschließend durch den Gasauslass des Sensors freigesetzt werden. Ist die Dihydroxybenzol-Verbindung beispielsweise 1,2,-Dihydroxybenzol, so können an der Arbeitselektrode die folgenden beiden Reaktionen ablaufen:

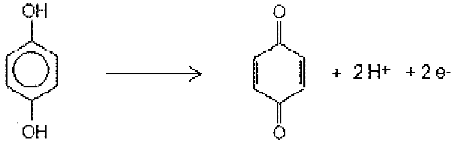

2NH₃ + 2H⁺ → 2NH₄⁺

An der Gegenelektrode können dann die beiden Rückreaktionen stattfinden, nämlich:

2 NH₄⁺ → 2NH₃+2H⁺

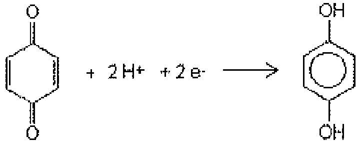

Im Rahmen dieser Reaktionen kann sich ein pH-Gradient zwischen der Arbeitselektrode und der Gegenelektrode bilden. Man erkennt, dass es daher günstig ist, wenn der Elektrolyt einen Puffer enthält, wobei der Puffer bevorzugt eine Verbindung entsprechend

Formel I R¹-(CR²R³)ₙ-SO₃H

mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(Hydroxyalkyl)alkyl). Ein solcher Puffer, besonders bevorzugt ein Puffer, bei welchem als R1 N-morpholino ausgewählt ist, insbesondere 3-(N-morpholino)propansulfonsäure oder eine andere 3-(N-morpholino)alkansulfonsäure, kann günstiger Weise zur Stabilisierung des pH-Wertes der Reaktionslösung dienen.

Mithin erkennt man, dass es vorteilhaft ist, wenn der Elektrolyt eine Zusammensetzung ist aus
a. einem Lösungsmittel, das ausgewählt ist aus Wasser, Propylencarbonat, Ethylencarbonat oder Gemische daraus,
b. einem Leitsalz, das ausgewählt ist aus 1-Hexyl-3-methylimidazolium-tris(pentafluoroethyl)trifluorophosphat oder Tetraalkylammoniumtoluolsulfat,
c. einem organischen Mediator, der ausgewählt ist aus der Gruppe enthaltend substituiertes Anthrachinon, substituiertes 1,2-Hydrochinon, subsituiertes 1,4-Hydrochinon, besonders bevorzugt tert-butyl-Hydrochinon oder Anthrachinon-2-Sulfonsäure.
d. optional einem Puffer, der ausgewählt ist aus 3-(N-morpholino)-propansulfonsäure oder 3-(N-morpholino)-ethansulfonsäure,
e. optional einer den Gefrierpunkt der Zusammensetzung erniedrigenden Verbindung, ausgewählt aus der Gruppe enthaltend Propylenglykol, Ethylenglykol.

Eine bevorzugte Ausführungsform eines erfindungsgemäßen elektrochemischen Gassensors kann insofern ein elektrochemischer Gassensor mit einem Gehäuse, einer Arbeitselektrode, einer Gegenelektrode und einer Referenzelektrode sein, wobei das Gehäuse ein Elektrolytreservoir, eine Gaseintrittsöffnung und wenigstens eine Gasaustrittsöffnung aufweist und das Elektrolytreservoir mit einem flüssigen Elektrolyten gefüllt ist, wobei der Elektrolyt wenigstens ein Lösungsmittel, ein Leitsalz und/oder einen organischen Mediator enthält und wobei der Elektrolyt eine Zusammensetzung ist aus
a. einem Lösungsmittel, das ausgewählt ist aus Wasser, Propylencarbonat, Ethylencarbonat oder Gemische daraus,
b. einem Leitsalz, das ausgewählt ist aus 1-Hexyl-3-methylimidazolium-tris(pentafluoraethyl)trifluorophosphat oder Tetraalkylammoniumtoluolsulfat.
c. einem organischen Mediator, der ausgewählt ist aus der Gruppe enthaltend substituiertes Anthrachinon, substituiertes 1,2-Hydrochinon, subsituiertes 1,4-Hydrochinon, besonders bevorzugt tert-butyl-Hydrochinon oder Anthrachinon-2-Sulfonsäure,
d. optional einem Puffer, der ausgewählt ist aus 3-(N-morpholino)-propansulfonsäure oder 3-(N-morpholino)-ethansulfonsäure,
e. optional einer den Gefrierpunkt der Zusammensetzung erniedrigenden Verbindung, ausgewählt aus der Gruppe enthaltend Propylenglykol, Ethylenglykol.

So ist beispielsweise ein elektrochemischer Gassensor denkbar, mit einem Gehäuse, einer Arbeitselektrode, einer Gegenelektrode und einer Referenzelektrode sein, wobei das Gehäuse ein Elektrolytreservoir, eine Gaseintrittsöffnung und wenigstens eine Gasaustrittsöffnung aufweist und das Elektrolytreservoir mit einem flüssigen Elektrolyten gefüllt ist, wobei der Elektrolyt wenigstens ein Lösungsmittel, ein Leitsalz und/oder einen organischen Mediator enthält und wobei der Elektrolyt eine Zusammensetzung ist aus einem Lösungsmittel, welches ein Gemische aus Propylencarbonat und Ethylencarbonat ist, 1-Hexyl-3-methylimidazolium-tris(pentafluoroethyl)trifluorophosphat als Leitsalz, tert-butyl-1,2-Dihydroxybenzol oder Anthrachinon-2-Sulfonsäure als organischem Mediator, sowie 3-(N-morpholino)-propansulfonsäure als Puffer. Bei einem solchen Gassensor ist es dann besonders vorteilhaft, wenn der Gassensor einen Gegenelektrodenträger aufweist, wobei die Gegenelektrode derart an dem Gegenelektrodenträger aufgehängt ist, dass sie im Elektrolytreservoir hängt und von allen Seiten von dem Elektrolyt umspült ist.

Man erkennt mithin, dass es bei einem flüssigen Elektrolyt für einen elektrochemischen Gassensor, insbesondere für einen elektrochemischen Gassensor, der zum Nachweis von NH₃ oder NH₃-haltigen Gasgemischen geeignet ist, günstig ist, wenn der Elektrolyt wenigstens ein Lösungsmittel, ein Leitsalz und/oder einen organischen Mediator enthält, wobei das Leitsalz eine ionische Flüssigkeit, ein anorganisches Salz, ein organisches Salz oder ein Gemisch daraus ist.

Insbesondere für elektrochemische Gassensoren, bei welchen Elektroden aus Edelmetall oder Kohlenstoffnanoröhren verwendet werden, kann ein solcher Elektrolyt mit großem Vorteil eingesetzt werden, um die Dauerbegasungsfestigkeit eines solchen Sensors zu verbessern. Insbesondere das Risiko einer wie oben beschriebenen Vergiftung kann auf diese Weise deutlich minimiert werden.

Dabei ist es von besonderem Vorteil, wenn der Elektrolyt einen Puffer enthält, wobei der Puffer bevorzugt eine Verbindung entsprechend

Formel I R¹-(CR²R³)ₙ-SO₃H

mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(Hydroxyalkyl)alkyl. Beispielsweise können R² und R³ unabhängig voneinander ausgewählt sein aus H, NH und OH, wobei n = 2 ist und R¹ ausgewählt ist aus der Gruppe enthaltend Piperazinyl, substituiertes Piperazinyl, N-morpholino, Cylcoalkyl, tris-(Hydroxyalkyl)alkyl. Vorstellbar ist zum Beispiel auch, dass R² und R³ unabhängig voneinander ausgewählt sind aus H, NH und OH, wobei n = 2 ist und R¹ ausgewählt ist aus der Gruppe enthaltend N-morpholino und tris-(Hydroxyalkyl)alkyl). Beispielsweise ist es dabei ganz besonders von Vorteil, wenn n = 2 oder n = 3 ist, wobei alle R² und R³ unabhängig voneinander ausgewählt sind aus H, NH und OH, und wobei R¹ ausgewählt ist aus [4-(2-Hydroxyethyl)-1]-piperazinyl, (N-morpholino), N-Cyclohexyl, tris-(Hydroxymethyl)methyl. Ganz besonders bevorzugt ist der Puffer 3-(N-morpholino)-propansulfonsäure oder 3-(N-morpholino)ethansulfonsäure. So ist es zum Beispiel denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, und wobei der Elektrolyt außerdem einen Puffer enthält, insbesondere einen Puffer, der ausgewählt ist aus 3-(N-morpholino)-propansulfonsäure oder 3-(N-morpholino)-ethansulfortsäure.

Um zu verhindern, dass der Elektrolyt nach einer gewissen Zeit austrocknet - z.B. wenn der Sensor im Dauerbetrieb verwendet werden soll - ist es außerdem vorteilhaft, wenn der Elektrolyt als weitere Komponente eine Komponente zur Erniedrigung des Dampfdruckes enthält. Dabei kann die weitere Komponente bevorzugt ein Alkylenglykol oder Polyalkylenglykol sein, besonderes bevorzugt Propylenglykol, Ethylenglykol oder ein Gemisch aus Propylenglykol und Ethylenglykol ist. So ist es zum Beispiel denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist und wobei der Elektrolyt außerdem wenigstens ein Alkylenglykol enthält, insbesondere ein Alkylenglyfcal, das ausgewählt ist aus Propylenglykol, Ethylenglylcol oder einem Gemisch aus Proylenglykol und Ethylenglykol.

Weiterhin ist es günstig, wenn das Lösungsmittel ausgewählt ist aus der Gruppe enthaltend Wasser und Alkylencarbonat oder Gemische daraus, bevorzugt ausgewählt aus der Gruppe enthaltend Wasser, Propylencarbonat, Ethylencarbonat oder Gemische daraus. Denkbar ist beispielsweise, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist und wobei das Lösungsmittel Wasser ist. Alternativ ist auch vorstellbar, dass der Elektrolyt ein Gemisch aus einem Lösungsmittel, einem Leitsalz und/oder einen organischen Mediator ist, wobei das Leitsalz eine ionische Flüssigkeit, ein anorganisches Salz, ein organisches Salz oder ein Gemisch daraus ist und wobei das Lösungsmittel Alkylencarbonat, insbesondere Propylencarbonat, Ethylencarbonat oder ein Gemisch aus Propylencarbonat und Ethylencarbonat ist. Dabei ist es insbesondere auch vorstellbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, wobei der Elektrolyt außerdem einen Puffer enthält, insbesondere einen Puffer, der ausgewählt ist aus 3-(N-morpholino)-propansulfonsäure oder 3-(N-morpholino)-ethansulfonsäure und wobei das Lösungsmittel Alkylencarbonat, insbesondere Propylencarbonat, Ethylencarbonat oder ein Gemisch aus Propylencarbonat und Ethylencarbonat ist. Außerdem ist es denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, wobei der Elektrolyt außerdem wenigstens ein Alkylenglykol enthält, insbesondere ein Alkylenglykol, das ausgewählt ist aus Propylenglykol, Ethylenglykol oder einem Gemisch aus Propylenglykol und Ethylenglykol, und wobei Lösungsmittel Alkylencarbonat, insbesondere Propylencarbonat, Ethylencarbonat oder ein Gemisch aus Propylencarbonat und Ethylencarbonat ist.

Bevorzugt ist das Anion des Leitsalzes ausgewählt aus der Gruppe enthaltend Halogenide, Carbonat, Sulfonat, Phosphat und/oder Phosphonat, bevorzugt ein Anion ausgewählt aus der Gruppe enthaltend Alkyl-sulfonat, Alkenyl-Sulfonat, Aryl-Sulfonat, Alkyl-phosphat, Alkenyl-Phosphat, Aryl-Phosphat, substituiertes Alkyl-sulfonat, substituiertes Alkenyl-sulfonat, substituiertes Aryl-sulfonat, substituiertes Alkyl-phosphat, substituiertes Alkenyl-phosphat,

substituiertes Aryl-phosphat, halogeniertes Phosphat, halogeniertes Sulfonat halogeniertes Alkyl-sulfonat, halogeniertes Alkenyl-sulfonat, halogeniertes Aryl-sulfonat, halogeniertes Alkyl-phosphat, halogeniertes Alkenyl-phosphat, halogeniertes Aryl-phosphat, besonders bevorzugt ein Anion ausgewählt aus der Gruppe enthaltend Fluorophosphat, Alkylfluorophosphat, Aryl-sulfonat, ganz besonders bevorzugt aus der Gruppe enthaltend Perfluoralkylfluorophosphat, Toluolsulfonat.

Dabei ist es vorteilhaft, wenn das Leitsalz als Kationen Metallionen, Oniumionen oder ein Gemisch aus Metallionen und Oniumionen enthält. Zum Beispiel können die Metallionen ausgewählt sein aus Alkalimetallionen oder Erdalkalimetallionen, bevorzugt aus Li, K und/oder Na. Günstig ist es, wenn die Oniumionen ausgewählt sind aus Ammonium-, Phosphonium, Guanidiniumkationen und heterocyclischen Kationen, bevorzugt ausgewählt aus AlkylAmmonium- und heterocyclischen Kationen, besonders bevorzugt ausgewählt aus AlkylAmmonium, Imidazolium und/oder substituierten Imidazoliumionen, wobei die substituierten Imidazolium-Ionen bevorzugt eine Struktur entsprechend 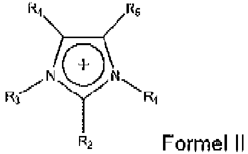 aufweisen, wobei R1, R2, R3, R4 und R5 unabhängig voneinander ausgewählt sein können aus -H, geradkettiges oder verzweigtes Alkyl mit 1 bis 20 C-Atomen, geradkettiges oder verzweigtes Alkenyl mit 2 bis 20 C-Atomen und einer oder mehreren Doppelbindungen, geradkettiges oder verzweigtes Aklinyl mit 2 bis 20 C-Atomen und einer oder mehreren Dreifachbindungen, gesättigtes, teilweise oder vollständig ungesättigtes Cycloalkyl mit 3-7 C-Atomen, das mit Alkylgruppen mit 1 bis 6C-Atomen substituiert sein kann, gesättigtes teilweise oder vollständig ungesättigtes Heteroaryl, Heteroaryl-C1-C6-alkyl oder Aryl-C1-C6-alkyl, wobei besonders bevorzugt R2, R4 und R5 H sind und R1 und R3 jeweils unabhängig voneinander ein geradkettiges oder verzweigtes Alkyl mit 1 bis 20 C-Atomen.

Beispielsweise ist insbesondere vorstellbar, dass als Leitsalz Tetrabutlyammoniumtoluolsulfonat oder 1-Hexyl-3-methyl-imidazolium-tris(pentafluoroethyl)-trifluorophosphat verwendet wird. Alternativ ist auch vorstellbar, dass das Leitsalz zum Beispiel LiCl, KCl oder eine Mischung aus LiCl und KCl ist. So ist es insbesondere vorteilhaft, wenn der Elektrolyt ein Gemisch aus einem Lösungsmittel, einem Leitsalz und/oder einem organischen Mediator ist, wobei das Leitsalz ausgewählt ist aus LiCl, KCl, Alkylammonium-toluolsulfonat und ionischen Flüssigkeiten, mit einem Perfluoralkylfluorophosphat-Anion.

Weiterhin ist es günstig, wenn der organische Mediator eine Polyhydroxyverbindung ist, welche bei Oxidation ein chinoides System oder ein Naphthalin-System bildet. Beispielsweise kann der organische Mediator ausgewählt sein aus der Gruppe enthaltend ortho-Dihydroxybenzol, para-Dihydroxybenzol, substituierte artho-Dihydroxybenzole und substituierte para-Dihydroxybenzole, Dihydroxynaphtalin, substituiertes Dihydroxynaphtalin, Anthrahydrochinon, substituiertes Anthrahydrochinon, bevorzugt 1,2-Dihydroxybenzol, 1,4 Dihydroxybenzol, Naphtohydrochinon, substituiertes 1,2- oder 1,4-Dihydroxybenzol, substituiertes Hydrochinon, substituiertes Naphtohydrochinon, besonders bevorzugt substituiertes Anthrahydrochinon, substituiertes Hydrochinon, substituiertes 1,2-Dihydroxybenzol. Dabei ist es besonders günstig, wenn die Substituenten des substituierten Anthrachinons, substituierten 1,2-Dihydroxybenzol und/oder substituierten 1,4-Hydrochinon ausgewählt sind aus der Gruppe enthaltend Sulfonyl, tert-Butyl, Hydroxy, Alkyl, Aryl, bevorzugt Sulfonsäure und/oder tert-Butyl.

In jedem Fall ist es besonders günstig, wenn der Elektrolyt als Lösungsmittel ein Gemisch aus Propylencarbonat und/oder Ethylencarbonat aufweist, als Leitsalz LiCl, KCl. Tetrabutylammoniumtoluolsulfonat und/oder 1-Hexyl-3-methyl-imidazolium-tris(pentafluoroethyl)-trifluorophosphat oder ein Gemisch aus zwei oder mehr dieser Komponenten aufweist und als organischen Mediator tert-butyl-Hydrochinon und/oder ein substituiertes Anthrachinon, bevorzugt Anthrachinon-2-Sulfonat aufweist.

Die Konzentration des organischen Mediators kann dabei zwischen 10⁻⁶ mol/ und 10⁻² ml/l liegen. So kann der organische Mediator in einer Konzentration von 10⁻² mol/l oder weniger, bevorzugt von 10⁻³ mol/l oder weniger, besonders bevorzugt von 5*10⁻⁴ mol/l oder weniger, ganz besonders bevorzugt von 2*10⁻⁴ mol/l oder weniger, ganz besonders bevorzugt von 10⁻⁴ mol/l oder weniger in dem Elektrolyt enthalten sein. Denkbar ist auch, dass der organische Mediator in einer Konzentration von 10⁻⁶ mol/l oder mehr, bevorzugt von 10⁻⁵ mol/l oder mehr, besonders bevorzugt von 5*10⁻⁵ mol/l oder mehr, ganz besonders bevorzugt von 8*10⁻⁵ mol/l oder mehr, ganz besonders bevorzugt von 10⁻⁴ mol/l oder mehr in dem Elektrolyt enthalten ist.
Insbesondere ist es auch denkbar, dass der organische Mediator in einer Konzentration von 10⁻⁵ mol/l bis 10⁻³ mol/l, bevorzugt 5*10⁻⁵ mol/l bis 5*10⁻⁴ mol/l, besonders bevorzugt 8*10⁻⁵ mol/l bis 2*10⁻⁴ mol/l, ganz besonders bevorzugt 10⁻⁴ mol/l vorhanden ist. In einem weiteren Aspekt, weist der Elektrolyt wenigstens ein Leitsalz, ein Lösungsmittel und einen organischen Mediator auf, wobei der Elektrolyt weiterhin einen Puffer aufweist, wobei der Puffer bevorzugt eine Verbindung entsprechend

Formel I R¹-(CR²R³)ₙ-SO₃H

mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(hydroxyalkyl)alkyl), wobei das Leitsalz eine ionische Flüssigkeit aufweist und wobei das Lösungsmittel ausgewählt ist aus Wasser, Propylencarbonat, Ethylencarbonat oder Gemische daraus. Bevorzugt ist dabei der organische Mediator ausgewählt aus der Gruppe enthaltend ortho-Chinone, para-Chinone, substituierte ortho-Chinone und substituierte para-Chinone, Dihydroxynaphatlin, substituiertes Dihydroxynaphtalin, Anthrachinon, substituiertes Anthrachinon, bevorzugt 1,2-Dihydroxybenzol, 1,4-Dihydroxybenzol, 1,4-Naphtodihydroxybenzol, substituiertes 1,2-Dihydroxybenzol, substituiertes 1,4-Dihydroxybenzol, substituiertes 1,4-Naphtodihydroxybenzol,
besonders bevorzugt substituiertes Anthrachinon, substituiertes 1,2-Dihydroxybenzol, subsituiertes 1,4-Dihydroxybenzol,
wobei die Substituenten des substituierten Anthrachinons, substituierten 1,2-Hydrochinon und/oder substituierten 1,4-Hydrochinon ausgewählt sind aus der Gruppe enthaltend Sulfonyl, tert-Butyl, Hydroxy, Alkyl, Aryl, bevorzugt Sulfonsäure, tert-Butyl.
Weiterhin bevorzugt ist dabei der Puffer eine Verbindung entsprechend

Formel I R¹-(CR²R³)ₙ-SO₃H

mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(hydroxyalkyl)alkyl), wobei der Puffer bevorzugt eine Verbindung entsprechend Formel I ist, mit n = 2 oder n = 3, wobei alle R² und R³ unabhängig voneinander ausgewählt sind aus H, NH und OH, und wobei R1 ausgewählt ist aus [4-(2-Hydroxyethyl)-1)-piperazinyl, (N-morpholino)-, N-Cyclohexyl-, tris-(hydroxymethyl)methyl, wobei der Puffer besonders bevorzugt 3-(N-morpholino)propansulfonsäure ist

Man erkennt, dass es besonders vorteilhaft ist, einen solchen flüssigen Elektrolyten in einem erfindungsgemäßen elektrochemischen Gassensor zu verwenden. Dabei ist insbesondere die Verwendung eines erfindungsgemäßen elektrochemischen Gassensors zum Nachweis von NH₃ oder NH₃-haltigen Gasgemischen günstig. Denkbar ist auch die Verwendung zum Nachweis von Amin-Verbindungen oder Hydrazin. Ebenfalls günstig ist mithin die Verwendung eines erfindungsgemäßen flüssigen Elektrolyten zum Nachweis von NH3 oder NH3-haltigen Gasgemischen. Auch hier ist selbstverständlich auch die Verwendung zum Nachweis von Amin-Verbindungen oder Hydrazin vorstellbar. Ganz besonders bevorzugt ist in diesem Zusammenhang auch die Verwendung eines erfindungsgemäßen elektrochemischen Gassensors, der einen erfindungsgemäßen flüssigen Elektrolyten enthält, zum Nachweis von NH₃ oder NH₃-haltigen Gasgemischen, Amin-Verbindungen und/oder Hydrazin.

Weitere Merkmale, Details und Einzelheiten ergeben sich aus den nachfolgend beschriebenen Figuren und Ausführungsbeispielen. Es versteht sich von selbst, dass diese Ausführungsbeispiele nur exemplarisch sind und dass sich für den Fachmann ohne Probleme weitere Varianten und Ausführungsbeispiele ergeben. Dabei zeigt
- Fig. 1: einen schematischen Aufbau eines erfindungsgemäßen elektrochemischen Gassensors.
- Fig 2a: einen schematischen Aufbau eines weiteren Ausführungsbeispiels eines erfindungsgemäßen Gassensors.
- Fig. 2b: eine Aufsicht auf das Trennelement des Gassensors aus Fig. 2a entlang der Schnittebene A-A
- Fig. 3a: einen schematischen Aufbau eines weiteren Ausführungsbeispiels eines erfindungsgemäßen Gassensors
- Fig. 3b: eine Aufsicht auf das Trennelement des Gassensors aus Fig. 2b entlang der Schnittebene C-C
- Fig.4: einen schematischen Ablauf einer Nachweisreaktion für NH₃ in einem elektrochemischen Gassensor, welcher einen erfindungsgemäßen Elektrolyten enthält.

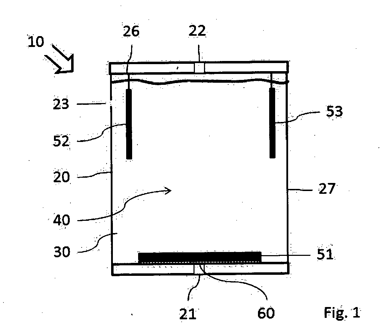

Man erkennt in Fig. 1 einen besonders einfachen Aufbau eines erfindungsgemäßen elektrochemischen Gassensors 10. Der Gassensor 10 hat ein Gehäuse 20 mit einem Gaseinlass 21 und einem Gasauslass 22. Das Gehäuse 20 hat weiterhin einen zusätzliche Öffnung 23, die zum Druckausgleich dient. Das Innere des Gehäuses 20 ist als Elektrolytreservoir 30 ausgebildet. In dem Gehäuse 20 sind eine Arbeitselektrode 51, eine Gegenelektrode 52 und eine Referenzelektrode 53 angeordnet.

Die Arbeitselektrode 51 ist hinter dem Gaseinlass 21 angeordnet, so dass sie mit einströmendem Gas, welches reaktive Spezien, enthält reagieren kann. Zwischen der Arbeitselektrode 51 und dem Gehäuse 20 ist im Bereich des Gaseinlasses 21 eine Schutzmembran 60 angeordnet.

Die Gegenelektrode 52 ist an einem Gegenelektrodenträger 26 aufgehängt. Der Gegenelektrodenträger 26 ist in diesem Ausführungsbeispiel am Gehäuse 20 befestigt. Man erkennt, dass die Gegenelektrode 52 von allen Seiten von Elektrolyt 40 umspült ist.

Der Elektrolyt 40 ist in diesem Ausführungsbeispiel eine Zusammensetzung aus einem Lösungsmittel, einem Leitsalz, einem organischen Mediator und einem Puffer. Dabei ist der Elektrolyt beispielsweise eine Zusammensetzung aus einem Lösungsmittel, welches ein Gemische aus Propylencarbonat und Ethylencarbonat ist, 1-Hexyl-3-methylimidazolium-tris(pentafluoroethyl)trifluorophosphat als Leitsalz, tert-butyl-1,2-Dihydroxybenzol oder Anthrachinon-2-Sulfonsäure als organischem Mediator, sowie 3-(N-morpholino)-propansulfonsäure als Puffer.

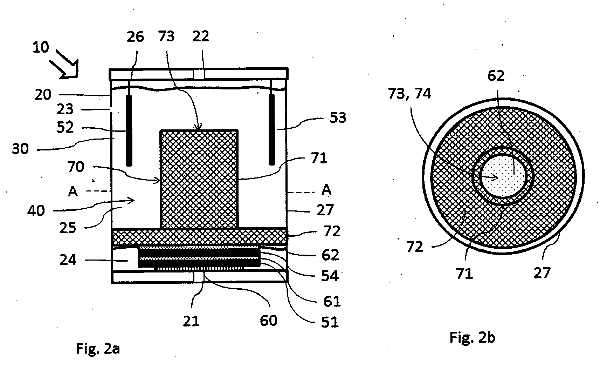

Auch der in Fig. 2a dargestellte erfindungsgemäße Gassensor 10 hat ein Gehäuse 20 mit einem Gaseinlass 21, einem Gasauslass 22 und einer zusätzlich Öffnung 23. In diesem Ausführungsbeispiel ist in dem Gehäuse 20 ein Trennelement 70 angeordnet, welches das Innere des Gehäuses 20 in eine untere Kammer 24 und eine obere Kammer 25 unterteilt. Die Gegenelektrode 52 ist wie in dem bereits in Fig. 1 beschriebenen Ausführungsbeispiel an einem Gegenelektrodenträger 26 befestigt und befindet sich in der oberen Kammer 25. Auch die Referenzelektrode 53 ist in der oberen Kammer 25 angeordnet.

Das Trennelement 70 besteht aus einem Oberteil 71 und einem Fußteil 72. Das Oberteil 71 ist röhrenförmig und hat eine obere Öffnung 73 und eine untere Öffnung (in Fig. 2a nicht dargestellt), die mit einer (ebenfalls in Fig. 2a nicht dargestellten) Öffnung des Fußteils 72 korrespondiert. Denkbar ist dabei auch, dass die untere Öffnung des Oberteils 71 der Öffnung des Fußteils 72 entspricht. Das Fußteil 72 erstreckt sich bis zur Gehäusewand 27 des Gehäuses 20. Auf diese Weise bildet das Fußteil 72 die Trennung zwischen der oberen und der unteren Kammer 24, 25. Die obere Kammer 25 bildet dabei das Elektrolytreservoir 30. Aus dem Elektrolytreservoir 30 kann der Elektrolyt 40 durch die obere Öffnung 73 des Oberteils 71 in das Trennelement 70 gelangen und von dort durch das Fußteil 72 hindurch in die untere Kammer 24 des Gehäuses fließen.

In der unteren Kammer 24 sind die Arbeitselektrode 51 und eine Abfangelektrode 54 angeordnet. Die Arbeitselektrode 51 ist dabei wie auch in dem in Fig. 1 dargestellten Ausführungsbeispiel derart hinter dem Gaseinlass 21 des Gehäuses 20 angeordnet, dass einströmendes Gas möglichst unmittelbar auf die Arbeitselektrode 51 trifft. Zwischen dem Gaseinlass 21 und der Arbeitselektrode 51 ist lediglich eine Schutzmembran 60 ausgebildet. Diese Schutzmembran 60 schützt die Arbeitselektrode 51 vor mechanischen Beschädigungen, die beispielsweise durch Staubteilchen entstehen können. Man erkennt, dass die Arbeitselektrode 51 von dem in die untere Kammer 24 einströmenden Elektrolyten 40 umspült ist.

Zwischen der Arbeitselektrode 51 und der Abfangelektrode 54 ist eine Zwischenmembran 61 angeordnet. Diese Zwischenmembran 61 ist derart formstabil, dass sie einen direkten Kontakt zwischen der Arbeitselektrode 51 und der Abfangelektrode 54 verhindert. Gleichzeitig ist die Zwischenmembran 61 mit dem Elektrolyten 40 getränkt. Auf diese Weise stehen die Arbeitselektrode 51 und die Abfangelektrode 54 in einem Fluidkontakt miteinander.

Auch zwischen der Abfangelektrode 54 und dem Fußteil 72 des Trennelementes 70 ist eine Zwischenmembran 62 angeordnet. Auch diese zweite Zwischenmembran 62 ist mit dem Elektrolyten 40 getränkt. Man erkennt, dass der Elektrolyt 40 auf diese Weise von der oberen Kammer 24 durch das Trennelement 70 und durch die Zwischenmembran 62 hindurch in die untere Kammer 25 strömt bzw. strömen kann. Dies wird auch insbesondere in dem in Fig. 2b dargestellten Querschnitt entlang der Linie A-A aus Fig. 2a deutlich. Man erkennt in diesem Querschnitt eine Aufsicht auf das in dem Gehäuse 20 angeordnete Trennelement 70. Das Fußteil 72 des Trennelementes 70 hat die Form einer Scheibe. Es erstreckt sich bis zur Gehäusewand 27. Das Oberteil 71 des Trennelementes 70 hat die Form eines Rohres mit einer oberen Öffnung 73 und einer unteren Öffnung 74. Die untere Öffnung 74 korrespondiert mit einer Öffnung im Fußteil 72. Dabei ist es durchaus auch denkbar, dass das Oberteil 71 und das Fußteil 72 einstückig, beispielsweise als durchgängiges Spritzguß- oder Drehteil ausgeführt sind, so dass die untere Öffnung des Oberteils 71 gleichzeitig die Öffnung des Fußteils 72 ist. In Fig. 2b blickt man nun entlang der Richtung B, die in Fig. 2a dargestellt ist, von oben auf das Trennelement 70. Durch das Oberteil 71 hindurch schaut man dabei auf die Zwischenmembran 62, die in der unteren Kammer 24 angeordnet ist.

In einem (nicht dargestellten) weiteren Ausführungsbeispiel ist es auch vorstellbar, dass bei einem Gassensor 10, der grundsätzlich wie der in Fig. 2a und 2b beschriebene Gassensor 10 ausgeführt ist, in der unteren Kammer 24 anstelle der Kombination aus Arbeitselektrode 51, Abfangelektrode 54 sowie erster und zweiter Zwischenmembran 61, 62 nur eine Arbeitselektrode 51 nicht aber eine Abfangelektrode 54 angeordnet ist. In diesem Fall ist die erste Zwischenmembran 61 direkt zwischen der Arbeitselektrode 51 und dem Fußteil 72 des Trennelementes 70 angeordnet.

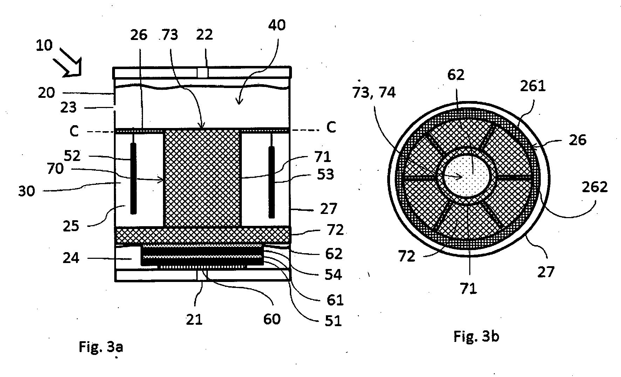

In den Fig. 3a und 3b erkennt man eine weitere Ausgestaltungsvariante des Trennelementes 70. Auch hier hat der Gassensor 10 - wie bereits in den vorangehend beschriebenen Ausführungsbeispielen - ein Gehäuse 20 mit einem Gaseinlass 21, einem Gasauslass 22, einer zusätzlich Öffnung 23 und einem Elektrolytreservoir 30. Das Trennelement 70 unterteil das Gehäuse 20 wiederum in eine obere und eine untere Kammer 24, 25. Das Elektrolytreservoir 30 wird auch hier durch die obere Kammer 24 gebildet. In der unteren Kammer sind die Arbeitselektrode 51, eine Abfangelektrode 54, sowie eine erste und eine zweite Zwischenmembran 61, 62 und eine Schutzmembran 60 wie bereits bei dem in Fig. 2a beschriebenen Ausführungsbeispiel angeordnet. Um Wiederholungen zu vermeiden, wird daher an dieser Stelle auf das bereits oben Ausgeführte verwiesen.

In dem in Fig. 3a und 3b dargestellten Ausführungsbeispiel ist der Gegenelektrodenträger 26 Teil des Trennelementes 70. Man erkennt, dass die Gegenelektrode 52 an diesem Gegenelektrodenträger 26 befestigt ist und in der oberen Kammer 25 angeordnet ist. Der Gegenelektrodenträger 26 ist wird dabei von wenigstens einer Speiche 261 gebildet, die radial vom Oberteil 71 des Trennelementes 70 zur Gehäusewand 27 des Gassensors 10 verläuft. Dabei hat der Gegenelektrodenträger 26 in dem dargestellten Beispiel einen äußeren Ring 262, der das Oberteil 71 in der Art eines Wagenrades umgibt. Die Speichen 261 verlaufen dabei von dem Oberteil 71 zum Ring 262. In einfacheren, alternativen (nicht dargestellten) Ausführungsbeispielen ist es jedoch auch vorstellbar, dass eine oder mehrere der Speichen 261 in direktem Kontakt mit der Gehäusewand 27 stehen, ohne dass ein solcher Ring 262 vorhanden ist.

Die Struktur des Gegenelektrodenträgers 26 erkennt man besonders gut in der in Fig. 3b gezeigten Aufsicht, die einem Querschnitt entlang der Linie C-C von Fig. 3a entspricht. Dabei blickt man in Richtung B (vgl. Fig. 2a) von oben auf das in dem Gassensor 10 angeordnete Trennelement 70.

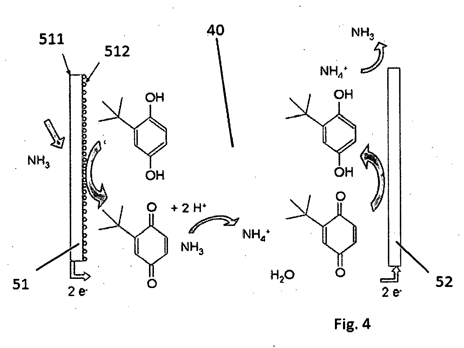

In jedem der beschriebenen Ausführungsbeispiele ist In dem Elektrolytreservoir 30 ein Elektrolyt 40 vorhanden. Dabei kann der Elektrolyt 40 sowohl zur Arbeitselektrode 51 als auch zur Abfangelektrode 54 gelangen. Ist die reaktive Spezies, wie in dem in Fig. 4 dargestellten Beispiel, NH₃ so kann jeweils eine chemische Reaktion zwischen einströmendem NH₃, dem Material der Arbeits- bzw. Abfangelektrode 51, 52 und dem Elektrolyten 40 stattfinden.

Dabei reagiert in den Gassensor 10 einströmendes NH₃ an der Oberfläche der Arbeitselektrode 51 mit dem Elektrolyten, Bevorzugt besteht die Arbeitselektrode 51 dabei z.B. aus einer PTFE-Membran 511 mit einer Kohlenstoff-Nanoröhren-Beschichtung 512. Die Gegenelektode 52 besteht bevorzugt aus einem Edelmetall. Der Elektrolyt 40 ist in diesem Beispiel eine Zusammensetzung aus Propylencarbonat und/oder Ethylencarbonat als Lösungsmittel, 1-Hexyl-3-methyl-imidazolium-tris(pentafluoroethyl)-trifluorophosphat als Leitsalz und tert-butyl-1, 2-Dihydroxybenzol als organischem Mediator. Der Elektrolyt enthält weiterhin bevorzugt einen Puffer, nämlich 3-(N-morpholino)propansulfonsäure. Wie in Fig. 4 erkennbar, wird das tert-Butyl-1,2-Dihydroxybenzol an der Arbeitselektode 51 zu tert-butyl-Chinon oxidiert. Die dabei freigesetzten Protonen reagieren mit dem in den Gassensor 10 einströmenden NH₃ zu Ammoniumionen. Die Ammoniumionen gelangen zur Gegenelektrode 52, wo die Rückreaktion des zuvor gebildeten tert-butyl-Chinon zu 1,2-Dihydroxybenzol stattfindet. Dabei wird aus den Ammoniumionen wiederum NH₃ freigesetzt, das durch den Gasauslass 22 entweichen kann. Im Zuge dieses Reaktionsablaufes stabilisiert der eingesetzte Puffer den pH-Wert des Elektrolyten, der zwischen der Arbeits- und der Gegenelektrode 51, 53 im Elektrolytreservoir 30 vorliegt. Man erkennt anhand der Fig. 4, dass ein Elektrolyt 40 zum Nachweis von Stickstoffhaltigen Verbindungen, insbesondere NH₃, verwendbar ist. Der Elektrolyt 40 ist dabei beispielsweise in einen Gassensor 10 entsprechend einem der Ausführungsbeispiele, die in den Fig. 1, 2a, 2b, 3a oder 3b gezeigt sind, in das Elektrolytreservoir 30 eingefüllt. Mit anderen Worten ein elektrochemischer Gassensor 10, wie er in den Fig. 1, 2a, 2b, 3a, 3b dargestellt ist kann insbesondere dann zum Nachweis von Stickstoffhaltigen Verbindungen, wie z.B. NH₃, Amin-Verbindungen und/oder Hydrazin verwendet werden, wenn in seinem Elektrolytreservoir 30 der oben beschriebene Elektrolyt 40 eingefüllt ist. Dabei ist es selbstverständlich, dass der Elektrolyt 40 nicht auf das in Bezug auf Fig. 1 beschriebene Beispiel beschränkt ist, sondern dass der Elektrolyt 40 jegliche Zusammensetzung sein kann, wie sie oben beschrieben ist.

**Bezugszeichenliste**

| | | | |
|---|---|---|---|
| A | Schnittebene | | |
| B | Richtung | | |
| C | Schnittebene | | |
| | | | |
| 10 | Gassensor | 511 | PTFE-Membran |
| 20 | Gehäuse | 512 | Kohlenstoff-Nanoröhren-Beschichtung |
| 21 | Gaseinlass | 52 | Gegenelektrode |
| 22 | Gasauslass | 53 | Referenzelektrode |
| 23 | Öffnung | 54 | Abfangelektrode |
| 24 | untere Kammer | 60 | Schutzmembran |
| 25 | obere Kammer | 61 | Zwischenmembran |
| 26 | Gegenelektrodenträger | 62 | Zwischenmembran |
| 261 | Speiche | 70 | Trennelement |
| 262 | Ring | 71 | Oberteil |
| 27 | Gehäusewand | 72 | Fußteil |
| 30 | Elektrolytreservoir | 73 | Öffnung |
| 40 | Elektrolyt | 74 | Öffnung |
| 51 | Arbeitselektrode | | |

## Patentansprüche

1. Elektrochemischer Gassensor (10) mit einem Gehäuse (20), einer Arbeitselektrode (51), einer Gegenelektrode (52) und einer Referenzelektrode (53), wobei das Gehäuse (20) ein Elektrolytreservoir (30), eine Gaseintrittsöffnung (21) und wenigstens eine Gasaustrittsöffnung (22) aufweist und wobei das Elektrolytreservoir (30) mit einem flüssigen Elektrolyten (40) gefüllt ist, , **dadurch gekennzeichnet, dass** der Gassensor (10) einen Gegenelektrodenträger (26) aufweist, wobei die Gegenelektrode (52) derart an dem Gegenelektrodenträger (26) aufgehängt ist, dass sie im Elektrolytreservoir (30) hängt und von allen Seiten von dem Elektrolyt (40) umspült ist.

2. Gassensor entsprechend Anspruch 1, **dadurch gekennzeichnet, dass** der Gassensor (10) ein Trennelement (70) aufweist, welches das Gehäuse (20) in eine obere Kammer (25) und eine untere Kammer (24) unterteilt.

3. Gassensor entsprechend Anspruch 2, **dadurch gekennzeichnet, dass** die obere Kammer (25) das Elektrolytreservoir (30) bildet.

4. Gassensor entsprechend Anspruch 2 oder 3, **dadurch gekennzeichnet, dass** die Arbeitselektrode (51) in der unteren Kammer (24) angeordnet ist.

5. Gassensor entsprechend wenigstens einem der Ansprüche 2 bis 4, **dadurch gekennzeichnet, dass** das Trennelement (70) derart ausgebildet ist, dass Elektrolyt (40) durch das Trennelement (70) hindurch aus der oberen Kammer (24) in die untere Kammer (25) leitbar ist.

6. Gassensor entsprechend wenigstens einem der Ansprüche 2 bis 5, **dadurch gekennzeichnet, dass** das Trennelement (70) wenigstens ein Oberteil (71) und ein Fußteil (72) aufweist.

7. Gassensor entsprechend wenigstens einem der Ansprüche 2 bis 6, **dadurch gekennzeichnet, dass** der Gegenelektrodenträger (26) ein Teil des Trennelementes (70) ist.

8. Gassensor entsprechend wenigstens einem der Ansprüche 1 bis 7, **dadurch gekennzeichnet, dass** der Gassensor (10) außerdem eine Abfangelektrode (54) aufweist.

9. Gassensor entsprechend wenigstens einem der Ansprüche 1 bis 8, **dadurch gekennzeichnet, dass** der Elektrolyt (40) wenigstens ein Lösungsmittel, ein Leitsalz und/oder einen organischen Mediator enthält.

10. Gassensor entsprechend Anspruch 9, **dadurch gekennzeichnet, dass** das Lösungsmittel ausgewählt ist aus der Gruppe enthaltend Wasser und Alkylencarbonat oder Gemische daraus, bevorzugt ausgewählt aus der Gruppe enthaltend Wasser, Propylencarbonat, Ethylencarbonat oder Gemische daraus.

11. Gassensor entsprechend wenigstens einem der Ansprüche 9 oder 10, **dadurch gekennzeichnet, dass** das Leitsalz eine ionische Flüssigkeit, ein anorganisches Salz oder ein Gemisch aus einer ionischen Flüssigkeit und einem anorganischen Salz ist, wobei das Anion bevorzugt ausgewählt ist aus der Gruppe enthaltend Halogenide, Carbonat, Sulfonat, Phosphat und/oder Phosphonat und wobei das Kation bevorzugt ausgewählt ist aus der Gruppe enthaltend Metallionen, Oniumionen oder ein Gemisch aus Metallionen und Oniumionen.

12. Gassensor entsprechend wenigstens einem der Ansprüche 9 bis 11, **dadurch gekennzeichnet, dass** der organische Mediator ein chinoides System aufweist, bevorzugt ausgewählt aus der Gruppe enthaltend ortho-Chinone, para-Chinone, substituierte ortho-Chinone und substituierte para-Chinone, Dihydroxynaphatlin, substituiertes Dihydroxynaphtalin, Anthrachinon, substituiertes Anthrachinon, besonders bevorzugt ausgewählt aus der Gruppe enthaltend 1,2-Dihydroxybenzol, 1,4-Dihydroxybenzol, 1,4-Naphtodihydroxybenzol, substituiertes 1,2-Dihydroxybenzol, substituiertes 1,4-Dihydroxybenzol, substituiertes 1,4-Naphtodihydroxybenzol, ganz besonders bevorzugt ausgewählt aus der Gruppe enthaltend substituiertes Anthrachinon, substituiertes 1,2-Dihydroxybenzol, subsituiertes 1,4-Dihydroxybenzol.

13. Gassensor entsprechend wenigstens einem der Ansprüche 9 bis 12, **dadurch gekennzeichnet, dass** der Elektrolyt einen Puffer enthält, wobei der Puffer bevorzugt eine Verbindung entsprechend
Formel I R¹-(CR²R³)ₙ-SO₃H
mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(hydroxyalkyl)alkyl).

14. Gassensor entsprechend wenigstens einem der Ansprüche 9 bis 13, **dadurch gekennzeichnet, dass** der Elektrolyt eine Zusammensetzung ist aus
a. einem Lösungsmittel, das ausgewählt ist aus Wasser, Propylencarbonat, Ethylencarbonat oder Gemische daraus,
b. einem Leitsalz, das ausgewählt ist aus 1-Hexyl-3-methylimidazolium-tris(pentafluoroethyl)trifluorophosphat oder Tetraalkylammoniumtoluolsulfat,
c. einem organischen Mediator, der ausgewählt ist aus der Gruppe enthaltend substituiertes Anthrachinon, substituiertes 1,2-Hydrochinon, subsituiertes 1,4-Hydrochinon, besonders bevorzugt tert-butyl-Hydrochinon oder Anthrachinon-2-Sulfonsäure
d. optional einem Puffer, der ausgewählt ist aus
e. optional einer den Gefrierpunkt der Zusammensetzung erniedrigenden Verbindung, ausgewählt aus der Gruppe enthaltend Propylenglykol, Ethylenglykol.

15. Verwendung eines elektrochemischen Gassensors gemäß einem der Ansprüche 1 bis 14 zum Nachweis von NH₃ oder NH₃-haltigen Gasgemischen.

## Claims

1. An electrochemical gas sensor (10) having a housing (20), a working electrode (51), a counter-electrode (52) and a reference electrode (53), wherein the housing (20) has an electrolyte reservoir (30), a gas-inlet opening (21) and at least one gas-outlet opening (22), and wherein the electrolyte reservoir (30) is filled with a liquid electrolyte (40), **characterised in that** the gas sensor (10) has a counter-electrode carrier (26), wherein the counter-electrode (52) is suspended on the counter-electrode carrier (26) in such a way that it is suspended in the electrolyte reservoir (30), and the electrolyte (40) flows around it on all sides.

2. A gas sensor according to claim 1, **characterised in that** the gas sensor (10) has a separating element (70) which divides the housing (20) into an upper chamber (25) and a lower chamber (24).

3. A gas sensor according to claim 2, **characterised in that** the upper chamber (25) forms the electrolyte reservoir (30).

4. A gas sensor according to claim 2 or 3, **characterised in that** the working electrode (51) is arranged in the lower chamber (24).

5. A gas sensor according to at least one of claims 2 to 4, **characterised in that** the separating element (70) is configured in such a way that electrolyte (40) can be directed through the separating element (70) out of the upper chamber (25) into the lower chamber (24).

6. A gas sensor according to at least one of claims 2 to 5, **characterised in that** the separating element (70) has at least one upper portion (71) and one foot portion (72).

7. A gas sensor according to at least one of claims 2 to 6, **characterised in that** the counter-electrode carrier (26) is a portion of the separating element (70).

8. A gas sensor according to at least one of claims 1 to 7, **characterised in that** the gas sensor (10) has, moreover, a collecting electrode (54).

9. A gas sensor according to at least one of claims 1 to 8, **characterised in that** the electrolyte (40) contains at least one solvent, a conducting salt and/or an organic mediator.

10. A gas sensor according to claim 9, **characterised in that** the solvent is selected from the group containing water and alkylene carbonate or mixtures thereof, preferably selected from the group containing water, propylene carbonate, ethylene carbonate or mixtures thereof.

11. A gas sensor according to at least one of claims 9 or 10, **characterised in that** the conducting salt is an ionic liquid, an inorganic salt or a mixture of an ionic liquid and an inorganic salt, wherein the anion is preferably selected from the group containing halides, carbonate, sulphonate, phosphate and/or phosphonate, and wherein the cation is preferably selected from the group containing metal ions, onium ions or a mixture of metal ions and onium ions.

12. A gas sensor according to at least one of claims 9 to 11, **characterised in that** the organic mediator has a quinoid system, preferably selected from the group containing ortho-quinones, para-quinones, substituted ortho-quinones and substituted para-quinones, dihydroxynaphthalene, substituted dihydroxynaphthalene, anthraquinone, substituted anthraquinone, particularly preferably selected from the group containing 1,2-dihydroxybenzene, 1,4-dihydroxybenzene, 1,4-naphthodihydroxybenzene, substituted 1,2-dihydroxybenzene, substituted 1,4-dihydroxybenzene, substituted 1,4-naphthodihydroxybenzene, especially preferably selected from the group containing substituted anthraquinone, substituted 1,2-dihydroxybenzene, substituted 1,4-dihydroxybenzene.

13. A gas sensor according to at least one of claims 9 to 12, **characterised in that** the electrolyte contains a buffer, wherein the buffer is preferably a compound according to
Formula I R¹-(CR²R³)ₙ-SO₃H
with n = 1, 2, 3, 4 or 5, preferably n = 2 or n = 3, wherein all R² and R³ are selected independently of one another from H, NH and OH, and wherein R¹ is selected from the group containing piperazinyl, substituted piperazinyl, N-morpholino, cycloalkyl, tris-(hydroxyalkyl)alkyl.

14. A gas sensor according to at least one of claims 9 to 13, **characterised in that** the electrolyte is a composition of
a. a solvent, which is selected from water, propylene carbonate, ethylene carbonate or mixtures thereof,
b. a conducting salt, which is selected from 1-hexyl-3-methylimidazolium-tris(pentafluoroethyl)trifluorophosphate or tetraalkylammonium toluene sulphate,
c. an organic mediator, which is selected from the group containing substituted anthraquinone, substituted 1,2-hydroquinone, substituted 1,4-hydroquinone, particularly preferably tert-butyl-hydroquinone or anthraquinone-2-sulphonic acid,
d. optionally a buffer, which is selected from
e. optionally a compound lowering the freezing point of the composition, selected from the group containing propylene glycol, ethylene glycol.

15. Use of an electrochemical gas sensor in accordance with one of claims 1 to 14 for the detection of NH₃ or NH₃-containing gas mixtures.

## Revendications

1. Capteur de gaz électrochimique (10) avec un boîtier (20), une électrode de travail (51), une contre-électrode (52) et une électrode de référence (53), le boîtier (20) comprenant un réservoir d'électrolyte (30), une ouverture d'entrée de gaz (21) et au moins une ouverture de sortie de gaz (22), et le réservoir d'électrolyte (30) étant rempli avec un électrolyte liquide (40), **caractérisé en ce que** le capteur de gaz (10) comprend un support de contre-électrode (26), la contre-électrode (52) étant suspendue au support de contre-électrode (26) de manière à pendre dans le réservoir d'électrolyte (30) et à baigner de tous les côtés dans l'électrolyte (40).

2. Capteur de gaz selon la revendication 1, **caractérisé en ce que** le capteur de gaz (10) comprend un élément de séparation (70), qui divise le boîtier (20) en une chambre supérieure (25) et une chambre inférieure (24).

3. Capteur de gaz selon la revendication 2, **caractérisé en ce que** la chambre supérieure (25) forme le réservoir d'électrolyte (30).

4. Capteur de gaz selon la revendication 2 ou 3, **caractérisé en ce que** l'électrode de travail (51) est agencée dans la chambre inférieure (24).

5. Capteur de gaz selon au moins l'une quelconque des revendications 2 à 4, **caractérisé en ce que** l'élément de séparation (70) est configuré de sorte que l'électrolyte (40) puisse être conduit au travers de l'élément de séparation (70) depuis la chambre supérieure (24) dans la chambre inférieure (25).

6. Capteur de gaz selon au moins l'une quelconque des revendications 2 à 5, **caractérisé en ce que** l'élément de séparation (70) comprend au moins une partie supérieure (71) et une partie inférieure (72).

7. Capteur de gaz selon au moins l'une quelconque des revendications 2 à 6, **caractérisé en ce que** le support de contre-électrode (26) est une partie ou pièce de l'élément de séparation (70).

8. Capteur de gaz selon au moins l'une quelconque des revendications 1 à 7, **caractérisé en ce que** le capteur de gaz (10) comprend en outre une électrode de capture ou d'interception (54).

9. Capteur de gaz selon au moins l'une quelconque des revendications 1 à 8, **caractérisé en ce que** l'électrolyte (40) contient au moins un solvant, un sel conducteur et/ou un médiateur organique.

10. Capteur de gaz selon la revendication 9, **caractérisé en ce que** le solvant est choisi dans le groupe contenant l'eau et un carbonate d'alkylène ou leurs mélanges, de préférence choisi dans le groupe contenant l'eau, le carbonate de propylène, le carbonate d'éthylène ou leurs mélanges.

11. Capteur de gaz selon au moins l'une quelconque des revendications 9 ou 10, **caractérisé en ce que** le sel conducteur est un liquide ionique, un sel inorganique ou un mélange d'un liquide ionique et d'un sel inorganique, l'anion étant de préférence choisi dans le groupe contenant les halogénures, les carbonates, les sulfonates, les phosphates et/ou les phosphonates, et le cation étant de préférence choisi dans le groupe contenant les ions métalliques, les ions onium et un mélange d'ions metalliques et d'ions onium.

12. Capteur de gaz selon au moins l'une quelconque des revendications 9 à 11, **caractérisé en ce que** le médiateur organique comprend un système quinoïde, de préférence choisi dans le groupe contenant les ortho-quinones, les para-quinones, les ortho-quinones substituées et les para-quinones substituées, la dihydroxynaphtaline, la dihydroxynaphtaline substituée, l'anthraquinone, l'anthraquinone substituée, de manière particulierèment préférée choisi dans le groupe contenant le 1,2-dihydroxybenzène, le 1,4-dihydroxybenzène, le 1,4-naphtodihydroxybenzène, le 1,2-dihydroxybenzène substitué, le 1,4-dihydroxybenzène substitué, le 1,4-naphtodihydroxybenzène substitué, de manière tout particulièrement préférée choisi dans le groupe contenant l'anthraquinone substituée, le 1,2-dihydroxybenzène substitué, le 1,4-dihydroxybenzène substituté.

13. Capteur de gaz selon au moins l'une quelconque des revendications 9 à 12, **caractérisé en ce que** l'électrolyte contient un tampon, le tampon correspondant de préférence à un composé
Formule I R¹-(CR²R³)ₙ-SO₃H
avec n = 1, 2, 3, 4 ou 5, de préférence n = 2 ou n = 3, tous les R² et R³ étant choisis indépendamment les uns des autres parmi H, NH et OH, et R¹ étant choisi dans le groupe contenant pipérazinyle, pipérazinyle substitué, N-morpholino, cycloalkyle, tris-(hydroxyalkyl)alkyle.

14. Capteur de gaz selon au moins l'une quelconque des revendications 9 à 13, **caractérisé en ce que** l'électrolyte est une composition formée par
a. un solvant, qui est choisi parmi l'eau, le carbonate de propylène, le carbonate d'éthylène ou leurs mélanges,
b. un sel conducteur, qui est choisi parmi le tris(pentafluoroéthyl)trifluorophosphate de 1-hexyl-3-méthylimidazolium ou le toluene-sulfate de tétraalkylammonium,
c. un médiateur organique, qui est choisi dans le groupe contenant l'anthraquinone substituée, la 1,2-hydroquinone substituée, la 1,4-hydroquinone substituée, de manière particulièrement préférée la tert-butyl-hydroquinone ou l'acide anthraquinone-2-sulfonique,
d. éventuellement un tampon, qui est choisi parmi
e. éventuellement un composé abaissant le point de congélation de la composition, choisi dans le groupe contenant le propylène glycol, l'éthylène glycol.

15. Utilisation d'un capteur de gaz électrochimique selon l'une quelconque des revendications 1 à 14 pour la détection de NH₃ ou de mélanges gazeux contenant du NH₃.
